# EUROPÄISCHE PATENTSCHRIFT

(11) **EP 2 799 092 B1**
(45) Veröffentlichungstag und Bekanntmachung des Hinweises auf die Patenterteilung: **02.12.2015**
(21) Anmeldenummer: 14165999.5
(22) Anmeldetag: 25.04.2014
(51) Int. Cl.: A61L 2/10, G01N 33/00, G02B 21/00

(54) **Lebendzellmikroskop mit UV-Licht-Sterilisator**
Live cell microscope with UV-light steriliser
Microscope pour cellules vivantes avec stérilisateur à la lumière UV

(30) Priorität: 30.04.2013 DE 102013007463
(43) Veröffentlichungstag der Anmeldung: 05.11.2014
(73) Patentinhaber: Carl Zeiss Microscopy GmbH, 07745 Jena (DE)
(72) Erfinder: Plohnke, Tim, 37085 Göttingen (DE); Scheps, Alexander, 37139 Adelebsen (DE); Rojahn, Timo, 37170 Uslar (DE); KIinge, Tobias, 37079 Göttingen (DE)
(74) Vertreter: Scholze, Humbert

(56) Entgegenhaltungen:
- EP-A2- 2 416 346
- CN-U- 202 570 197
- DATABASE WPI Week 199050 Thomson Scientific, London, GB; AN 1990-368579 XP002729198, & BR 9 000 203 A (MAZURKEVICIUS H R) 13. November 1990 (1990-11-13)

## Beschreibung

Die Erfindung betrifft ein automatisiertes Lebendzellmikroskop, mit einem im Gehäuse des Mikroskops angeordneten Probenhalterahmen, einer ultraviolettes Licht mit einer Wellenlänge von 280 nm bis 100 nm (UV-C Strahlung) emittierenden Sterilisationseinheit sowie mit Befestigungs-, Bedien- und Steuerelementen zum Zwecke der Fixierung und Bewegungseinleitung des Probenhalterahmens sowie der Sterilisationseinheit.

Um in der Lebendzellmikroskopie korrekte Ergebnisse zu erzielen, ist es zwingend notwendig, sicherzustellen, dass die Proben in einem sterilen Umfeld bearbeitet und beobachtet werden.

An konventionellen Forschungsmikroskopen ist es üblich die mit der Probe in Verbindung kommenden Bauteile durch Wischen mit Lösemitteln oder Autoklavieren zu desinfizieren. Zudem kann auch das gesamte Mikroskop einer UV-C-Strahlung, das heißt einer ultravioletten Strahlung mit einer Wellenlänge von 280 bis 100 nm, ausgesetzt werden.

In der automatisierten Mikroskopie funktionieren diese Methoden durch bauliche Unterschiede zu den konventionellen Mikroskopen nur bedingt. Automatisierte Mikroskope sind häufig als geschlossene Systeme aufgebaut und unter der Bezeichnung "Box-Mikroskop" bekannt. Hier ist es mühsam und zeitaufwändig an die innenliegenden Bauräume zu gelangen um sie zu sterilisieren. Automatisieren lassen sich die bisher bekannten Methoden nicht.

Eine bekannte Lösung ist das "MagNa STARLet" welches in der Produktbroschüre der Hamilton Robotics Ltd beschrieben wird. In diesem Liquid-Handling-Automaten sind fest installierte UV-C abstrahlende Niederdruckgasentladungsröhren installiert. Diese sorgen für eine stets sterile Umgebung. Aus Platzgründen lässt sich diese Einrichtung schwer umsetzen.

Des Weiteren wird in der Patentschrift WO 98/20108 ein Verfahren zur Autoklavierung eines Probenraums beschrieben. Nachteilig hierbei ist, dass der Probenraum auseinandergebaut werden muss, um an die zu reinigenden Komponenten zu gelangen. Dies ist ein zeitaufwendiger und für eine automatisierte Lebendzellmikroskopie nicht zumutbarer Prozess.

Ausgehend von den Nachteilen der Lösungen des Standes der Technik liegt der Erfindung die Aufgabe zugrunde, ein automatisiertes Lebendzellmikroskop dahingehend weiter zu bilden, dass in einem möglichst kleinen zu inkubierenden Bauraum stabile Umgebungsbedingungen gewährleistet werden.

Diese Aufgabe wird mit einem Lebendzellmikroskop der eingangs beschriebenen Art durch die Merkmale des Patentanspruchs 1 gelöst. Vorteilhafte Ausgestaltungen sind in den Unteransprüchen 2 bis 14 angegeben.

Erfindungsgemäß ist die UV-C Sterilisationseinheit im Lebendzellmikroskop zum Zwecke der Sterilisation den Proberaum durchfahrbar angeordnet.

Denkbar wäre dazu, dass die UV-C Sterilisationseinheit in einer Art Parkposition fest im automatisierten Lebendzellmikroskop untergebracht ist und zum Zwecke der Sterilisation in den Probenraum eingeführt werden kann. Ebenso könnte auch eine UV-C Sterilisationseinheit in den vorhandenen Probenhalterahmen eingesetzt werden.

In einer vorteilhaften Ausgestaltung ist die Sterilisationseinheit als Wechselelement ausgebildet, wobei im Gehäuse des Lebendzellmikroskops ein Wechselbereich zur wahlweisen Aufnahme des Probenhalterahmens oder der UV-C Sterilisationseinheit vorhanden ist. Dazu sind der Probenhalterahmen oder die UV-C Sterilisationseinheit an einem im Gehäuse des Lebendzellmikroskops befindlichen XY-Tisch angeordnet und können in den Wechselbereich automatisiert eingeführt werden. Vom Wechselbereich durchfahren entweder der Probenhalterahmen oder die UV-C Sterilisationseinheit den Probenraum bei verschiedenen Verfahrwegen (Verfahrmodi), während sie dann im Wechselbereich vom XY-Tisch wieder lösbar sind.

Vorteilhafterweise sind die Aufnahmekonturen zur Fixierung des Probenhalterahmens und der Sterilisationseinheit sowie deren Abmessungen so gestaltet, dass ein schneller Wechsel im Transferbereich möglich ist.

Bedingt durch die gleichen Abmessungen und den gleichen Bewegungsbereich von Probenhalterahmen und UV-C Sterilisationseinheit wird sichergestellt, dass eine vollständige Sterilisation durchgeführt wird.

In einer weiteren vorteilhaften Ausgestaltung erfolgt diese Fixierung des Probenhalterahmen oder der Sterilisationseinheit über Klemmverbindungen mit dem XY-Tisch, wobei die Aufnahmekonturen Öffnungen aufweisen, in die jeweils ein am XY-Tisch befestigter Exzenterhebel von einer waagerechten Klemmposition in eine senkrechte Freigabeposition eingreifbar ist.

Zweckmäßigerweise ist die Sterilisationseinheit mit Niederdruckgasentladungsröhren ausgestattet, wobei über eine Inverterschaltung die erforderliche Zünd- und Betriebsspannung erzeugt wird.

Ebenso denkbar wäre eine Bestückung mit anderen UV-C emittierenden Leuchtmitteln wie beispielsweise LEDs, Quecksilberdampflampen oder Quarz-Halogen-Lampen. Eine weitere denkbare Möglichkeit wäre es, die UV-C emittierenden Leuchtmittel in den Seitenwänden oder Boden und Deckel des Verfahrbereichs (Probenraumes) anzuordnen, wobei eine ortsfeste Leuchte über Spiegel im gesamten Verfahrbereich Licht streuen könnte.

Durch die Anordnung von Spiegeln, die es ermöglichen, jeden Winkel auszuleuchten, könnten Schattenbildungen verhindert werden.

Da an den Seitenwänden des Verfahrbereichs bauartbedingt Bereiche nicht ausreichend gut bestrahlt werden, wäre vor der Bewegung der UV-C Sterilisationseinheit im Verfahrbereich in XY-Richtung eine Bewegung in Z-Richtung denkbar.

Zum Zuführen der erforderlichen Zünd- und Betriebsspannung (24 Volt) sind vorteilhafterweise an der UV-C Sterilisationseinheit Kontakte vorhanden, die als Federkontakte ausgebildet sind.

Jeweils zwei Kontakte dienen dabei zur Erkennung des Probenhalterahmens und der UV-C Sterilisationseinheit und zwei Kontakte zur Spannungsversorgung der Niederdruckgasentladungsröhren.

Eine weitere vorteilhafte Ausgestaltung besteht darin, dass zum Zwecke der Verringerung von Absorptionsverlusten durch die tragende Mechanik im zu sterilisierenden Bereich das Gehäuse der UV-C Sterilisationseinheit eine Vielzahl von Aussparungen aufweist, wobei ohne Stabilitätsverlust Aussparungen relativ groß und Stege relativ klein gehalten sind.

Ferner ist es von Vorteil, wenn Mittel zur Veränderung der Geschwindigkeit der Bewegung der UV-C Sterilisationseinheit im Verfahrbereich des Probenraumes vorhanden sind. Dies ist dann erforderlich, wenn baulich bedingt der Abstand der UV-C Sterilisationseinheit zur Wand des Probenraumes unterschiedlich ist, oder es Bereiche gibt, die stärker verschmutzt sind. Diese Veränderung kann über eine entsprechende Programmierung automatisiert ablaufen oder vom Benutzer direkt vorgegeben werden.

Die erfindungsgemäße Einrichtung soll nachstehend anhand eines Ausführungsbeispiels näher erläutert werden. Dazu zeigen:
- Figur 1:: eine Darstellung des automatisierten Lebendzellmikroskops,
- Figur 2a:: eine Darstellung des Probenhalterahmens,
- Figur 2b:: eine Darstellung der UV-C Sterilisationseinheit,
- Figur 3:: eine Darstellung der Stromversorgung,
- Figur 4:: eine Darstellung des Transfer- und des Verfahrbereichs und
- Figur 5:: eine Darstellung der Verfahrmodi.

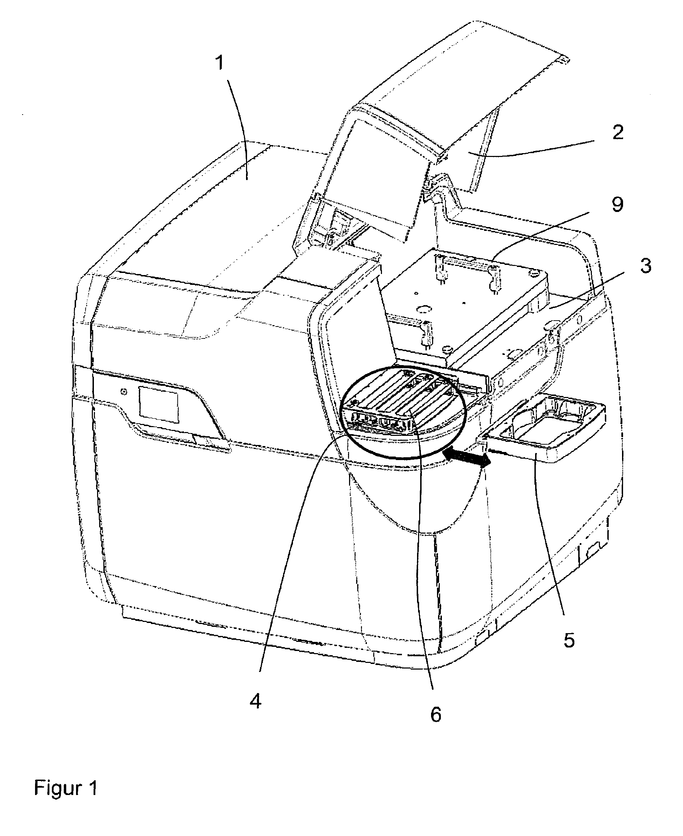

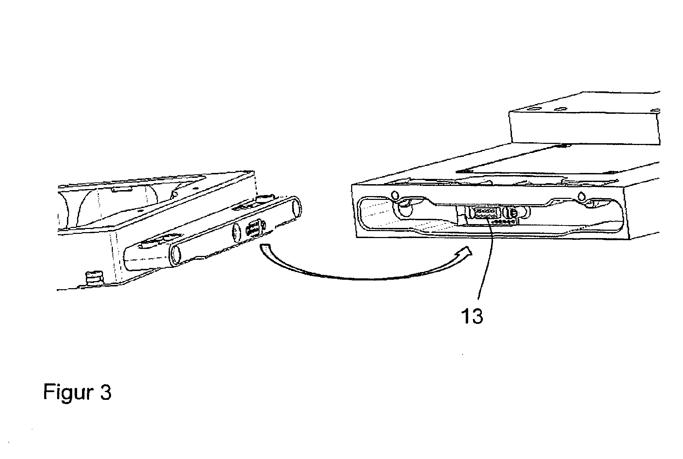

Figur 1 zeigt ein automatisiertes Lebendzellmikroskop, an dessen Gehäuse 1 eine Geräteklappe 2 zum Öffnen eines Probenraumes 3 vorhanden ist. Das Lebendzellmikroskop besitzt einen Wechselbereich 4 zur wahlweisen Aufnahme eines Probenhalterahmens 5 oder einer UV-C Sterilisationseinheit 6, die Licht mit einer Wellenlänge 280 nm bis 100 nm aussendet. Wie aus Figur 1 ersichtlich, befindet sich hier die UV-C Sterilisationseinheit 6 im Wechselbereich 4. Sowohl der Probenhalterahmen 5 als auch die UV-C Sterilisationseinheit 6 befinden sich auf einen XY-Tisch 7 (Figur 3) durchfahren nacheinander einen Transferbereich 8, einen definierten Verfahrbereich 9 im Probenraum 3, die in Figur 3 näher dargestellt sind, und gelangen dann wieder zum Zwecke des Wechselns in den Wechselbereich 4.

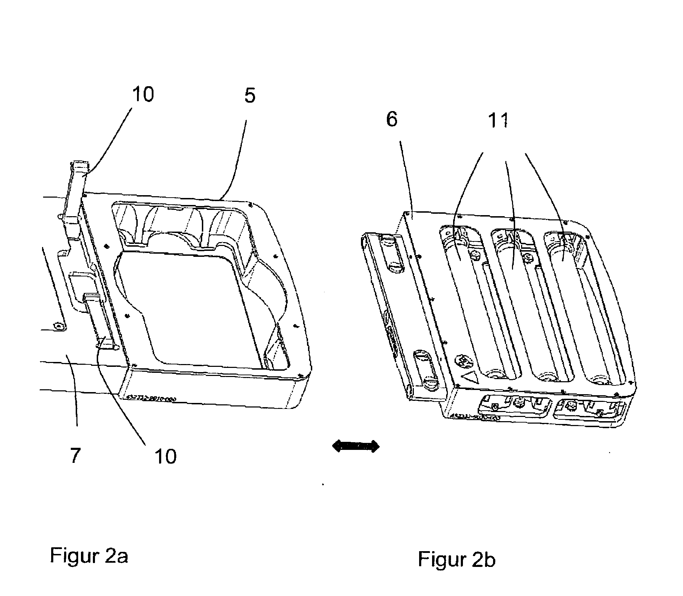

Figur 2a zeigt den Probenhalterahmen 5, der am XY-Tisch 7 über eine Klemmverbindung fixiert ist, wobei zwei am XY-Tisch 7 befestigte Exzenterhebel 10, durch entsprechende Öffnungen im Probehalterahmen 5 gehend, von einer senkrechten Freigabeposition in eine waagerechte Klemmposition gebracht werden. In den Freigabepositionen können der Probenhalterahmen 5 und die UV-C Sterilisationseinheit 6 seitlich aus dem Wechselbereich 4 herausgezogen werden.

Die gleichen Anschlusskonturen wie der Probenhalterahmen 5 besitzt die in Figur 2b dargestellte UV-C Sterilisationseinheit 6, die mit drei Niederdruckgasentladungsröhren 11 bestückt ist.

Zum Zwecke der automatisierten Erkennung des Probehalterrahmens 5 und der UV-C Sterilisationseinheit 6 sind am Probenhalterahmen 5 und an der UC-C Sterilisationseinheit 6 nicht näher dargestellte Federkontakte vorhanden, deren Impulse von einem ebenfalls nicht dargestellten, programmierbaren Microcontroller, beispielsweise von einem an sich bekannten "1-wire EPROM", erfassbar sind. Gleichzeitig wird über Federkontakte 13 die Stromversorgung gewährleistet (Figur 3).

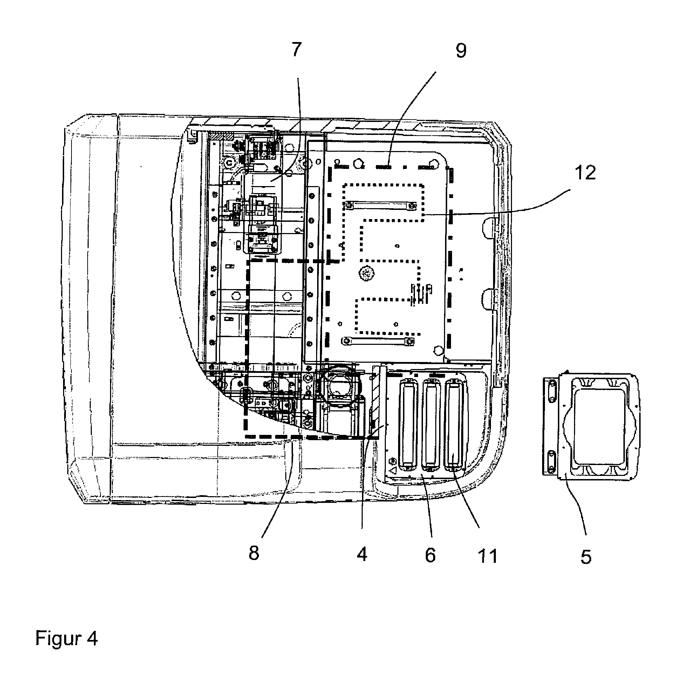

Figur 4 zeigt eine Darstellung des automatisierten Lebendzellmikroskop aus der Draufsicht mit dem XY-Tisch 7 zur wahlweisen Aufnahme des Probenhalterahmens 5 oder der UV-C Sterilisationseinheit 6, den Wechselbereich 4 den Transferbereich 8, den Verfahrbereich 9, Verfahrmodi 12 sowie die am XY-Tisch 7 befestigte UV-C Sterilisationseinheit 6. Der Probenhalterahmen 5 befindet sich hier in der Wartestellung.

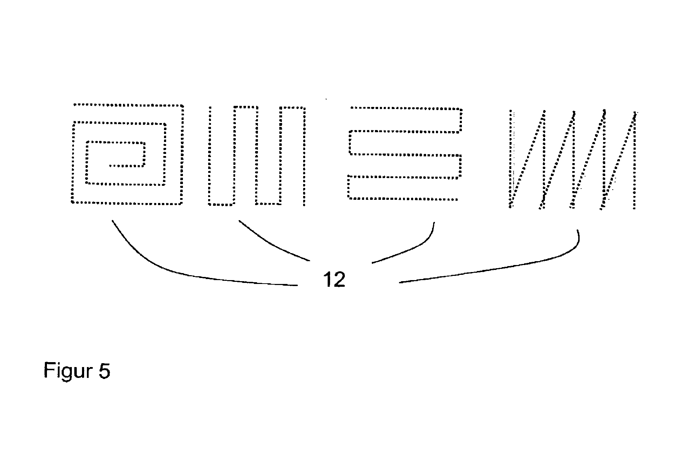

In Figur 5 sind verschiedene Verfahrmodi 12 vom Probenhalterahmen 5, beziehungsweise von der UV-C Sterilisationseinheit 6 zu sehen.

Nach der Fixierung im Wechselbereich 4 fährt der XY-Tisch 7 in die UV-C Sterilisationseinheit 6 über den Transferbereich 8 in den Verfahrbereich 9. Über zwei nicht dargestellte Federelemente erfolgt die Spannungsversorgung von 24 Volt, die über eine Inverterschaltung die für die Niederdruckgasentladungsröhren 11 erforderliche Zünd- und Betriebsspannung erzeugt. Die Spannung für die UV-C Sterilisationseinheit 6 wird durch eine Steuerung eingeschaltet, nachdem geprüft wurde, dass es sich bei der eingelegten Einheit tatsächlich um die UV-C Sterilisationseinheit 6 handelt. Ferner wird dabei geprüft, ob alle für den Prozess sicherheitsrelevanten Gehäuseöffnungen geschlossen sind.

Per Eingabe wir die abgeschlossene Fixierung der UV-C Sterilisationseinheit 6 im Wechselbereich 4 bestätigt. Die UV-C Sterilisationseinheit 6 wird hinter die letzte Verschlussblende gefahren, die sich anschließend schließt. Nach erfolgreichem Schließen der Verschlussblende erfolgt ein Signal, die UV-C Sterilisationseinheit 6 wird eingeschaltet und emittiert die UV-Strahlung.

Anschließend fährt die UV-C Sterilisationseinheit 6 in das Gehäuse 1 des Lebendzellmikroskops und bestrahlt entweder unter scannender Bewegung oder Schritt für Schritt den gesamtem im Lebendzellmikroskop liegenden Transferbereich 8 und Verfahrbereich 9.

Der Probenhalterahmen 5 kann in der Zwischenzeit auf konventionellem Weg autoklaviert werden. Die UV-C Sterilisationseinheit 6 ist durch großzügige Freifräsungen so gefertigt, dass sich die von den Niederdruckgasentladungsröhren 11 emittierte UV-C Strahlung unter möglichst geringen Absorptionsverlusten durch die tragende Mechanik im zu sterilisierenden Bereich (Probenraum 3) ausbreiten kann. Dies wird dadurch erreicht, dass die Aussparungen in der UV-C Sterilisationseinheit möglichst groß und die Stege möglichst klein sind, aber nicht die Stabilität beeinträchtigen. Eine technische Umsetzung ist in Figur 2b zu sehen.

Die Belichtungszeiten können in unterschiedlichen Bereichen variieren. Um den gesamten Verfahrbereich 9 abzuscannen können verschiedenste Modi 12 gefahren werden. Mögliche Varianten in XY-Richtung sind in der Figur 4 dargestellt.

Nachdem die vollständige Sterilisation erfolgt ist, fährt die UV-C Sterilisationseinheit 6 zurück in den Wechselbereich 4. Hier wird sie, wie bereits beschrieben, durch den Probenhalterahmen 5 ausgetauscht. Das automatisierte Lebendzellmikroskop kann anschließend wieder verwendet werden.

### Bezugszeichenliste

- 1: Gehäuse
- 2: Geräteklappe
- 3: Probenraum
- 4: Wechselbereich
- 5: Probenhalterahmen
- 6: UV-C Sterilisationseinheit
- 7: XY-Tisch
- 8: Transferbereich
- 9: Verfahrbereich
- 10: Exzenterhebel
- 11: Niederdruckgasentladungsröhre
- 12: Verfahrmodi
- 13: Federkontakte

## Patentansprüche

1. Automatisiertes Lebendzellmikroskop, mit einem im Gehäuse (1) des Mikroskops angeordneten Probenhalterahmen (5), mit einer Einrichtung zur Sterilisation eines Probenraums des Lebendzellmikroskops mit einer ultraviolettes Licht mit einer Wellenlänge von 280 nm bis 100 nm (UV-C Strahlung) emittierenden UV-C Sterilisationseinheit (6) sowie mit Befestigungs-, Bedien- und Steuerelementen zum Zwecke der Fixierung und Bewegungseinleitung des Probenhalterahmens (5), **dadurch gekennzeichnet, dass** die UV-C Sterilisationseinheit (6) im Lebendzellmikroskop zum Zwecke der Sterilisation den Proberaum (3) durchfahrbar angeordnet ist.

2. Automatisiertes Lebendzellmikroskop nach Anspruch 1, **dadurch gekennzeichnet, dass** die UV-C Sterilisationseinheit (6) im Lebendzellmikroskop integriert und zum Zwecke der Sterilisation in den Probenraum (3) einführbar ist.

3. Automatisiertes Lebendzellmikroskop nach Anspruch 1, **dadurch gekennzeichnet, dass** die UV-C Sterilisationseinheit (6) als Wechselelement ausgebildet ist, wobei im Gehäuse (1) des Lebendzellmikroskops ein Wechselbereich (4) zur wahlweisen Aufnahme des Probenhalterahmens (5) oder der UV-C Sterilisationseinheit (6) vorhanden ist, der Probenhalterahmen (5) oder die UV-C Sterilisationseinheit(6) auf einem im Gehäuse (1) des Lebendzellmikroskops befindlichen XY-Tisch (7) angeordnet, in den Wechselbereich (4) einführ-, den Probenraum (3) durchfahrend und im Wechselbereich (4) vom XY-Tisch (7) lösbar sind.

4. Automatisiertes Lebendzellmikroskop nach den Ansprüchen 1 und 3, dass Aufnahmekonturen zur Fixierung des Probenhalterahmens (5) und der UV-C Sterilisationseinheit (6) vorhanden sind, deren Abmessungen gleich sind.

5. Automatisiertes Lebendzellmikroskop nach Anspruch 3, **dadurch gekennzeichnet, dass** die Aufnahmekonturen über Klemmverbindungen mit dem XY-Tisch (7) verbunden sind.

6. Automatisiertes Lebendzellmikroskop nach Anspruch 3, **dadurch gekennzeichnet, dass** die Aufnahmekonturen Öffnungen aufweisen, in die jeweils ein am XY-Tisch (7) befestigter Exzenterhebel (10) von einer waagerechten Klemmposition in eine senkrechte Freigabeposition eingreifbar ist.

7. Automatisiertes Lebendzellmikroskop nach den Ansprüchen 1 und 2, **dadurch gekennzeichnet, dass** die UV-C Sterilisationseinheit mit Niederdruckgasentladungsröhren (11) ausgestattet ist.

8. Automatisiertes Lebendzellmikroskop nach den Ansprüchen 1 und 2, **dadurch gekennzeichnet, dass** zum Zwecke der Erkennung des Probehalterrahmens (5) und der UV-C Sterilisationseinheit (6) am Probenhalterahmen (5) und an der UV-C Sterilisationseinheit (6) Kontakte vorhanden sind, deren Impulse von einem programmierbaren Microcontroller erfassbar sind.

9. Automatisiertes Lebendzellmikroskop nach Anspruch 8, **dadurch gekennzeichnet, dass** als Microcontroller ein "1-wire EPROM" verwendet wird.

10. Automatisiertes Lebendzellmikroskop nach den Ansprüchen 1 und 2, **dadurch gekennzeichnet, dass** zum Zwecke der Zuführung der für die Niederdruckgasentladungsröhren (11) der UV-C Sterilisationseinheit (6) erforderlichen Zünd- und Betriebsspannung (24 Volt) Kontakte an der UV-C Sterilisationseinheit (6) vorhanden sind.

11. Automatisiertes Lebendzellmikroskop nach den Ansprüchen 8 und 10, **dadurch gekennzeichnet, dass** die Kontakte als Federkontakte ausgebildet sind, wobei jeweils zwei Kontakte zur Erkennung des Probenhalterahmens (5) und der UV-C Sterilisationseinheit (6) und zwei Kontakte zur Spannungsversorgung Niederdruckgasentladungsröhren (11) vorhanden sind, und zur Erzeugung der erforderlichen Zünd- und Betriebsspannung eine Inverterschaltung vorhanden ist.

12. Automatisiertes Lebendzellmikroskop nach den Ansprüchen 1 und 2, **dadurch gekennzeichnet, dass** zum Zwecke der Verringerung von Absorptionsverlusten durch die tragende Mechanik im zu sterilisierenden Bereich das Gehäuse (1) der UV-C Sterilisationseinheit (6) eine Vielzahl von Aussparungen aufweist, wobei ohne Stabilitätsverlust die Aussparungen relativ groß und die Stege relativ klein gehalten sind.

13. Automatisiertes Lebendzellmikroskop nach den Ansprüchen 1 und 2, **dadurch gekennzeichnet, dass** Mittel zur Veränderung der Geschwindigkeit der Bewegung der UV-C Sterilisationseinheit (6) im Verfahrbereich (9) des Probenraumes (3) vorhanden sind.

14. Automatisiertes Lebendzellmikroskop nach Anspruch 13, **dadurch gekennzeichnet, dass** die Betätigung der Mittel zur Veränderung der Geschwindigkeit der Bewegung der UV-C Sterilisationseinheit (6) automatisiert (programmiert) einstellbar oder vom Benutzer vorgebbar ist.

## Claims

1. Automated live cell microscope, comprising a sample holding frame (5) arranged in the housing (1) of the microscope, comprising an apparatus for sterilizing a sample space of the live cell microscope comprising a UV-C sterilization unit (6) emitting ultraviolet light with a wavelength of 280 nm to 100 nm (UV-C radiation) and comprising attachment, operating and control elements for fixing, and introducing the movement of, the sample holding frame (5), **characterized in that** the UV-C sterilization unit (6) is arranged in a manner traversable through the sample space (3) in the live cell microscope for the purposes of sterilization.

2. Automated live cell microscope according to Claim 1, **characterized in that** the UV-C sterilization unit (6) is integrated into the live cell microscope and insertable into the sample space (3) for the purposes of sterilization.

3. Automated live cell microscope according to Claim 1, **characterized in that** the UV-C sterilization unit (6) is embodied as an interchangeable element, wherein an interchange region (4) is present in the housing (1) of the live cell microscope for selectively receiving the sample holding frame (5) or the UV-C sterilization unit (6), the sample holding frame (5) or the UV-C sterilization unit (6) being arranged on an XY-stage (7) situated in the housing (1) of the live cell microscope, insertable into the interchange region (4), said sample holding frame or said UV-C sterilization unit traversing through the sample space (3) and being detachable from the XY-stage (7) in the interchange region (4).

4. Automated live cell microscope according to Claims 1 and 3, **characterized in that** receiving contours for fixing the sample holding frame (5) and the UV-C sterilization unit (6) are present, the dimensions of which are the same.

5. Automated live cell microscope according to Claim 3, **characterized in that** the receiving contours are connected to the XY-stage (7) by way of clamping connections.

6. Automated live cell microscope according to Claim 3, **characterized in that** the receiving contours have openings, into which respectively one eccentric lever (10), which is fastened to the XY-stage (7), can engage from a horizontal clamping position into a vertical release position.

7. Automated live cell microscope according to Claims 1 and 2, **characterized in that** the UV-C sterilization unit is equipped with low pressure gas discharge tubes (11).

8. Automated live cell microscope according to Claims 1 and 2, **characterized in that**, for the purposes of identifying the sample holding frame (5) and the UV-C sterilization unit (6), contacts are present at the sample holding frame (5) and at the UV-C sterilization unit (6), the pulses from which contacts are acquirable by a programmable microcontroller.

9. Automated live cell microscope according to Claim 8, **characterized in that** a "1-wire EPROM" is used as microcontroller.

10. Automated live cell microscope according to Claims 1 and 2, **characterized in that** contacts are present at the UV-C sterilization unit (6) for the purposes of supplying the ignition and operating voltage (24 volt) required for the low pressure gas discharge tubes (11) of the UV-C sterilization unit (6).

11. Automated live cell microscope according to Claims 8 and 10, **characterized in that** the contacts are respectively embodied as spring contacts, wherein there respectively are two contacts for identifying the sample holding frame (5) and the UV-C sterilization unit (6) and two contacts for supplying the voltage for the low pressure gas discharge tubes (11), and there is an inverter circuit for producing the required ignition and operating voltage.

12. Automated live cell microscope according to Claims 1 and 2, **characterized in that**, for the purposes of reducing absorption losses by the support mechanism, the housing (1) of the UV-C sterilization unit (6) has a number of cut-outs in the region to be sterilized, wherein, without loss of stability, the cut-outs are kept relatively large and the webs are kept relatively small.

13. Automated live cell microscope according to Claims 1 and 2, **characterized in that** means for changing the speed of movement of the UV-C sterilization unit (6) are present in the displacement region (9) of the sample space (3).

14. Automated live cell microscope according to Claim 13, **characterized in that** the actuation of the means for changing the speed of movement of the UV-C sterilization unit (6) is adjustable in an automated (programmed) manner or it is predeterminable by the user.

## Revendications

1. Microscope automatique d'observation de cellules vivantes, présentant
un bâti (5) de support d'échantillon disposé dans le boîtier (1) du microscope,
un dispositif de stérilisation d'un espace d'échantillon du microscope d'observation de cellules vivantes présentant une unité (6) de stérilisation par UV-C émettant une lumière ultraviolette d'une longueur d'onde de 280 nm à 100 nm (rayonnement UV-C),
ainsi que des éléments de commande et de contrôle qui permettent la fixation et l'introduction d'un déplacement du bâti (5) de support d'échantillon, **caractérisé en ce que**
l'unité (6) de stérilisation par UV-C est disposée dans le microscope d'observation de cellules vivantes de manière à pouvoir se déplacer dans l'espace d'échantillons (3) en vue de la stérilisation.

2. Microscope automatique d'observation de cellules vivantes selon la revendication 1, **caractérisé en ce que** l'unité (6) de stérilisation par UV-C est intégrée dans le microscope d'observation de cellules vivantes et peut être insérée dans l'espace (3) d'échantillon en vue de la stérilisation.

3. Microscope automatique d'observation de cellules vivantes selon la revendication 1, **caractérisé en ce que** l'unité (6) de stérilisation par UV-C est configurée comme élément remplaçable, une partie remplaçable (4) permettant de reprendre sélectivement le bâti (5) de support d'échantillon ou l'unité (6) de stérilisation par UV-C étant prévue dans le boîtier (1) du microscope d'observation de cellules vivantes, le bâti (5) de support d'échantillon ou l'unité (6) de stérilisation par UV-C étant disposés sur une table XY (7) située dans le boîtier (1) du microscope d'observation de cellules vivantes, pouvant être insérés dans la partie remplaçable (4) en traversant l'espace d'échantillon (3) et pouvant être séparés de la table XY (7) dans la partie remplaçable (4).

4. Microscope automatique d'observation de cellules vivantes selon les revendications 1 et 3, **caractérisé en ce que** des contours de reprise permettant de fixer le bâti (5) de support d'échantillon et l'unité (6) de stérilisation par UV-C étant prévus à des dimensions identiques.

5. Microscope automatique d'observation de cellules vivantes selon la revendication 3, **caractérisé en ce que** les contours de reprise sont raccordés à la table XY (7) par des liaisons serrées.

6. Microscope automatique d'observation de cellules vivantes selon la revendication 3, **caractérisé en ce que** les contours de réception présentent des ouvertures dans lesquelles un levier excentrique (10) fixé sur la table XY (7) peut être engagé depuis une position horizontale de serrage jusque dans une position verticale de libération.

7. Microscope automatique d'observation de cellules vivantes selon les revendications 1 et 2, **caractérisé en ce que** l'unité de stérilisation par UV-C est équipée de tubes (11) de décharge dans un gaz à basse pression.

8. Microscope automatique d'observation de cellules vivantes selon les revendications 1 et 2, **caractérisé en ce que** pour détecter le bâti (5) du support d'échantillon et l'unité (6) de stérilisation par UV-C, des contacts dont les impulsions peuvent être détectées par un microcontrôleur programmable sont prévus sur le bâti (5) du support d'échantillon et sur l'unité (6) de stérilisation par UV-C.

9. Microscope automatique d'observation de cellules vivantes selon la revendication 8, **caractérisé en ce qu'**une "EPROM à 1 fil" est utilisée comme microcontrôleur.

10. Microscope automatique d'observation de cellules vivantes selon les revendications 1 et 2, **caractérisé en ce que** pour amener la tension d'allumage et de service (24 volts) nécessaire pour les tubes (11) de décharge en gaz à basse pression de l'unité (6) de stérilisation par UV-C, des contacts sont prévus sur l'unité (6) de stérilisation par UV-C.

11. Microscope automatique d'observation de cellules vivantes selon les revendications 8 et 10, **caractérisé en ce que** les contacts sont configurés comme contacts élastiques, deux contacts étant prévus pour détecter le bâti (5) de support d'échantillon et l'unité (6) de stérilisation par UV-C et deux contacts étant prévus pour l'alimentation en tension des tubes (11) de décharge dans un gaz à basse pression, un circuit inverseur étant prévu pour former la tension nécessaire pour l'allumage et le service.

12. Microscope automatique d'observation de cellules vivantes selon les revendications 1 et 2, **caractérisé en ce que** pour diminuer les pertes par absorption par le mécanisme de support, le boîtier (1) de l'unité (6) de stérilisation par UV-C présente plusieurs découpes dans la partie à stériliser, les découpes étant relativement grandes et les traverses relativement petites sans perte de stabilité.

13. Microscope automatique d'observation de cellules vivantes selon les revendications 1 et 2, **caractérisé en ce que** des moyens de modification de la vitesse du déplacement de l'unité (6) de stérilisation par UV-C sont prévus dans la partie traversée (9) de l'espace (3) d'échantillon.

14. Microscope automatique d'observation de cellules vivantes selon la revendication 13, **caractérisé en ce que** l'actionnement des moyens de modification de la vitesse du déplacement de l'unité (6) de stérilisation par UV-C peut être réglé de manière automatique (ou programmée) ou peut être défini par l'utilisateur.
